# EUROPEAN PATENT SPECIFICATION

(11) **EP 0 811 845 B1**
(45) Date of publication and mention of the grant of the patent: **03.09.2003**
(21) Application number: 97303538.9
(22) Date of filing: 23.05.1997
(51) Int. Cl.: G01N 33/543, B01L 3/18, G01N 33/569

(54) **Devices and kits for testing serum and the like**
Vorrichtung und Testsatz zum Test von Serum und dergleichen
Dispositif et kit de test pour tester sérum et similaires

(30) Priority: 07.06.1996 EP 96304309
(43) Date of publication of application: 10.12.1997
(73) Proprietor: OXOID LIMITED, Basingstoke Hampshire RG24 8PW (GB)
(72) Inventor: Radcliffe, David Michael, Basingstoke, Hampshire RG21 3AN (GB); Head, Cheryl Mary Anne, Basingstoke, Hampshire RG26 5RF (GB)
(74) Representative: Lipscombe, Martin John

(56) References cited:
- EP-A- 0 056 563
- EP-A- 0 112 077
- EP-A- 0 165 001
- EP-A- 0 368 624
- EP-A- 0 391 109
- WO-A-96/01273
- US-A- 3 770 383

## Description

### Field of the invention

This invention relates to devices and kits for testing sample liquids such as serum.

### Background to the Invention

Agglutination assays have been widely used for many years in the testing of sample liquids. Traditionally such testing was conducted on a solid surface such as a glass microscope slide, and the presence of an agglutinated particulate reagent observed either with the naked eye or with the aid of a microscope. Today, assay kits are commercially available in which the particulate reagent is provided in dry form pre-dosed onto an appropriate solid surface, for example a "test card". The sample liquid is applied to the card, for example as a drop of serum. To conserve reagents and to facilitate handling of the test card without risk of contaminating the dried reagent, this reagent is usually located in a designated test area on the card, for example a clearly marked area of circular or other shape. It is usually necessary physically to agitate the sample liquid to encourage reconstitution of the dried particulate reagent. Thereafter the agglutination reaction can proceed and the test result can be evaluated by inspection of the test card after an appropriate time interval. Test devices of this type are taught, for example, in US 3,770,383.

To reassure the user that the test card is performing satisfactorily it is desirable for a control to be provided. Typically this is a positive control which assists the user to appreciate the degree of, agglutination (or its relative absence) indicative of a clearly positive test result. For example, in some assays this control is provided in the form of a liquid sample containing the analyte of interest, for example antibodies (e.g. in serum) raised against an infective agent of interest (such as *H*. *pylori* or *Neisseria*), or a preparation comprising antigens from the organism of interest (which may take the form of an antigen extract or a bacterial cell suspension). To maintain the integrity of this liquid control reagent it must be stored at low temperature. The need for access to the refrigerated storage facilities severely limits the practical situations under which such tests can be conducted.

Furthermore, in presently-available tests, reconstitution of the dried particulate reagent does not always occur effectively.

### General Description of the Invention

In a first aspect the invention provides an assay device for testing for the presence of an analyte in a sample liquid supported on a test surface, the device comprising a test stick having a handle portion to be held by a person performing the test and a mixing portion to contact the sample liquid, the mixing portion bearing a dried, pre-dosed reagent for the test, the reagent being deposited as a plurality of closely-spaced spots, such that contacting the mixing portion of the test stick with the sample liquid reconstitutes the test reagent, wherein the mixing portion is configured to provide a mixing surface to mix the

reconstituted test reagent with the sample liquid on the test surface. Preferably, the pre-dosed test reagent is particulate and the test results are revealed by the occurrence of, or inhibition of, agglutination of the particles following mixing with the sample liquid.

The test surface may be any solid surface on which an assay, such as an agglutination assay, can be conducted and visualized. Conventionally, such surfaces take the form of test cards or similar. It is essential that under normal usage conditions applied sample liquid should remain essentially on the surface of the card. Accordingly, the test card should not soak up the sample liquid. Plastics material, in sheet or moulded form, is ideal, but absorbent materials such as paper and cardboard can easily be used if the working surface is rendered non-absorbent by pre-treatment or lamination with non-absorbent material, such as polypropylene or cellulose acetate. The presently available test card materials can be used with the assay device of the invention without any difficulty. The test surface does not constitute part of the present invention.

Where the test result is based on an agglutination assay the pre-dosed dried reagent on the test stick should be particulate. Conventional agglutination assay reagents are entirely suitable. Preferably, these are "latex", e.g. polystyrene, particles bearing a specific binding agent, such as an antibody or antigen, which leads to agglutination of the particles during the test. Typically these particles have a size in the range of 200 to 1000 nm, more usually 300 to 500 nm. The particles should have a colour that is distinctly visible against the test card background. Usually the particles themselves are strongly coloured, e.g. blue or red, and used against a white or other light-coloured card background. However, white particles can be used against a dark, e.g. black, background. Erythrocytes or Protein A-bearing particles are conventional alternatives to latex particles in some tests. In the presence of a "positive" sample, agglutination may either be caused or inhibited. For the purposes of the invention there is no necessity for the agglutination reagent to differ from those conventionally used.

In contrast to existing procedures, advantages are obtained if the sample liquid is not applied directly onto the pre-dosed dried reagent. We have noted that direct drop-wise addition of the sample liquid onto a pre-dosed reagent can create problems of poor or uneven release of the dried material, and "clumping" of the dried material which can be confused with true agglutination. In accordance with the invention it is preferable to add the sample liquid to the test card (which is not pre-dosed with the dried reagent). After addition, the sample liquid is moved and mixed with the pre-dosed reagent by use of the assay device of the present invention, ensuring uniform and effective reconstitution of the dried reagent before the agglutination reaction commences.

Conveniently the test stick comprises plastics, card or similar material of sufficient rigidity to facilitate mixing of the test reagent and sample. In a preferred embodiment the test stick is provided as a substantially linear component (conveniently substantially planar, such as a spatula or the like), in which the mixing portion is joined to the handle portion by a region which is flexible (e.g. foldably deformable), such that the mixing portion can readily be bent out of the line of the stick to provide a surface (angled relative to the handle portion of the stick) which facilitates ease of mixing of the constituted test reagent and the sample liquid. This can readily be achieved by having a line of weakness, such as an impressed fold line or crease, perforation line, or a partial cut, running across the width of the stick close to one end. Simple finger pressure on the handle portion of the stick while the mixing portion is touching a surface, such as the test card, can cause the stick to flex at the line of weakness. The line of weakness in effect defines the boundary between the mixing portion and the handle portion.

Alternatively, the test stick may be formed of resiliently deformable material such that the stick can be bent during use to provide the mixing portion at an angle to the handle portion, thereby facilitating mixing of the sample with the test reagent.

Pre-dosing of the test reagent can readily be achieved using conventional reagent deposition technology such as reagent printing and micro-pipetting. Typically the total volume of deposited reagent will be from about 1 to about 50 µl, which may be applied as more than one spot. Precise positioning can be achieved using a X-Y plotter, for example. After deposition, the reagents can be dried by using conventional procedures. The provision of stabilising agents such as non-specific proteins, (e.g. BSA), and/or carbohydrates and sugars (such as sucrose, sorbitol or trehalose), is beneficial. A typical aqueous buffer may contain from about 1 to about 30 mg/ml BSA and from about 10 to about 100 mg/ml sucrose. These can be included in deposition buffers or added subsequently in accordance with conventional procedures. Typical buffers are phosphate buffer, usually at a pH of about 7, glycine buffer, Tris buffer and borate buffer.

The dry reagents of the invention can also incorporate additives that improve the performance of the assay, for example inhibitors for non-specific binding, e.g. a surfactant such as "Tween 20", and eliminators of interfering factors such as Rheumatoid-factor in serum. It will be appreciated that drying of reagents on the test stick increases the stability of the reagent (e.g. proteinaceous substances), especially at ambient temperatures.

It is preferred that at least the mixing portion (or the region thereof upon which the dried reagent is deposited), and desirably the whole test stick, is formed of a transparent or translucent material. This has the advantage that a person using the test stick in the course of an assay can readily determine visually when the dried reagent has been properly reconstituted and released from the test stick. A number of transparent or translucent plastics materials are known and which have properties suitable for making test sticks in accordance with the invention. These include polystyrene, polyethylene, polypropylene, polymers of acrylic or methacrylic acids, and related compounds and derivatives of the foregoing.

A further optional feature of the test stick is that it is configured so as to be capable of being used to collect bacterial colonies from the surface of agar plates. It is also a preferred feature that the test stick is capable of capturing a small volume of liquid (such as an aqueous sample liquid). Typically the liquid will be captured by a specially adapted region of the mixing portion, and desirably the liquid will be held separated from the dried reagent so as to prevent premature reconstitution thereof. Typically the liquid will be captured by surface tension and/or capillary action. For example, the liquid capture region may take the form of a small loop at one end region of the test stick (similar to those known for conventional inoculating loops), or may take the form of a narrow straight channel in the test stick.

The captured liquid may be deposited on the surface of a test card by touching the liquid capture region of the test stick onto the surface of the test card, so as to contact the captured liquid with the test card. Alternatively, the liquid capture region may be deformed by pressing against the test card, so as to release the captured liquid.

In certain embodiments it may be desirable to provide a plurality of substances on the test stick, each of the substances being formed as a discrete dried spot. For example, where substances are incompatible upon medium or long term storage together, they can be provided on the test stick as separate dried spots which only mix upon reconstitution during performance of the assay, such that they are sufficiently stable for the few minutes which it takes to perform and read a typical assay.

In a second aspect the invention provides a kit for testing for the presence of an analyte in a sample, the kit comprising the assay device defined above. More particularly, the kit will conveniently comprise a plurality of such assay devices. Preferably the plurality of test sticks will be provided as a "tear-off" array of separable, disposable sticks, preferably in a side-by-side arrangement, each stick being separated from adjacent sticks by a line of weakness (such as a fold, crease, partial cut line, perforation etc). In a preferred embodiment the dried test reagent comprises an antibody specific for a Lancefield group-specific antigen. Desirably, the kit will comprise a plurality of arrays of sticks, each array of sticks having an antibody specific for a different Lancefield group-specific antigen.

Other components may be included with advantage. Such optional components include a test card which provides a test surface to support the sample liquid during the assay. Preferably the test sticks and the test card (if provided) are disposable. Additionally, the Lancefield grouping agglutination assay, used to assign streptococci to one of the various Lancefield groups, requires an extraction step performed on the bacterial cells prior to the assay - in a kit for performing Lancefield grouping assays therefore, it is desirable to include reagents for performing the extraction (such as enzyme preparations or compounds for the *in situ* synthesis of nitrous acid).

It may also be preferred in certain embodiments for the kit to comprise one or more sticks provided with a control reagent, (which typically will give rise to a known result), thereby facilitating interpretation of the test results. Preferably the control reagent will be a positive control, but if desired a negative control stick may be provided instead of, or in addition to, the positive control stick. The control sticks, if included in the kit, are clearly marked, so as to be distinguishable from the test sticks.

In a third aspect, the invention provides a method of testing for the presence of an analyte in a sample liquid supported on a test surface, comprising using a test stick having a handle portion to be held by a person performing the test and a mixing portion bearing a dried, pre-dosed reagent for the test, such that the mixing portion of the test stick is contacted with the sample liquid so as to reconstitute the test reagent, and moving the mixing portion relative to the test surface so as to cause mixing of the reconstituted reagent with the sample liquid.

The invention can be applied in a wide range of assays. By way of example only, this range includes any assay conventionally conducted using the principle of particle agglutination. Such assays are routinely used to detect infective organisms such as H. *pylori, Neisseria*, Streptococci, *Legionella*, *E*. *coli*, *Salmonella* and *Staphylococcus*. Particularly preferred are kits for performing "Lancefield grouping" of Streptococci. Other examples of assays which may be conducted in accordance with the present invention include rapid biochemical tests for oxidase, in which tetramethyl-p-phenylene diamine dihydrochloride with ascorbic acid is used for differentiating oxidase-positive organisms e.g. *Neisseria gonorhoeae*, from oxidase-negative organisms (e.g. *E. coli*); and β-lactamase tests, which utilise Nitrocefin, a chromogenic substrate, for rapid detection of penicillinase-producing strains. In most cases the reagents would be dried on either a flat card and used in conjunction with a manipulation device such as a loop. Additional liquid such as water, saline solution or buffer solution will be required in some cases.

### Brief Description of the Drawing

Figure 1 shows an array of "tear-off" test stick assay devices in accordance with the invention.

Figure 2 shows one of the test sticks, separated from the remainder of the array, in use.

### Detailed Description of the Invention

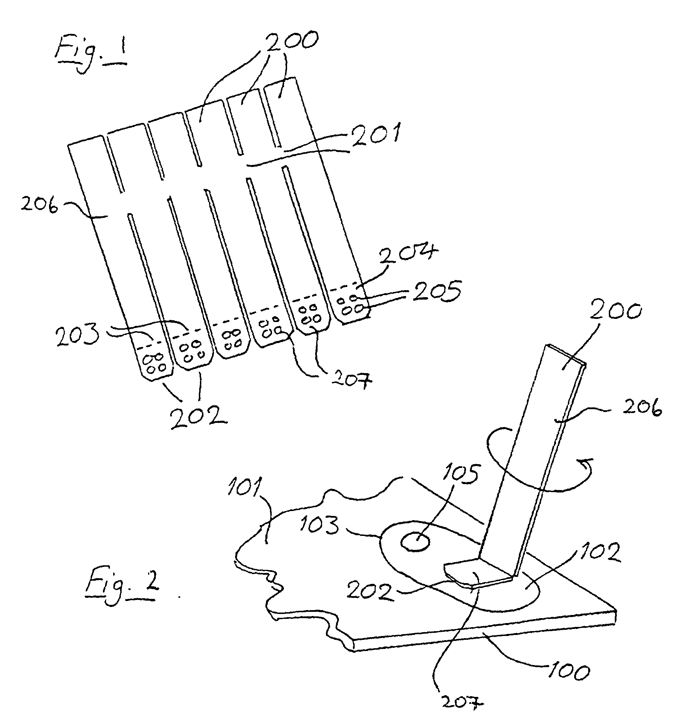

Referring to Figure 1, the test sticks 200 comprise a side-by-side arrangement of sticks which have been cut or stamped from a sheet of card. The cutting or stamping has left a small web 201 of card material joining each adjacent pair of sticks. The sticks 200 are therefore clearly distinct from one another, but are joined together in the array and readily spearated by hand from one another by tearing the residual web 201. The corners of one end 202 of each stick 200 are cut off so that this has distinctive shape. The end region 207 about end 202 of each stick 200 constitutes the mixing portion which is the working end of the stick in use. A short distance up the stick 200 from end 202 is a partial transverse cut 203 across the entire width of each stick, which ensures that the mixing portion 207 of the stick is easily bent out of alignment when the stick is pressed downwards against a solid surface, such as the surface of a test card. The region 206 constitutes the handle portion of each test stick 200.

Each stick 200 is about 40mm long and 11mm wide. The handle portion 206 is about 31mm long, the mixing portion about 11mm long.

The surface 204 of the mixing portion 202 which forms the underside of the mixing portion 207 when the stick 200 is bent, bears a dried pre-dosed test reagent 205. As depicted in Figure 1 this is deposited as four closely-spaced spots of reagent.

Figure 2 shows a test stick 200 in use. The stick 200 is in contact with a test card surface 101 and the stick mixing portion 207 is bent out of alignment with the handle portion 206 thereof. The stick 200 is being used in a scrubbing or stirring motion to move sample material and reagents within the test area 102 defined by line 103 on the test card 101.

### Examples

### Example 1

The following, which is given by way of example only, illustrates the manufacture of a test kit in accordance with the invention.

A *Helicobacter pylori* Latex Agglutination Test comprises a Dry Spot Latex reagent. The reagent consists of polystyrene latex particles of size 350 nm coated with a soluble extract of *H*. *pylori* cells. This is prepared in a phosphate buffer pH 7.3 which contains bovine serum albumin at a concentration of 10mg per ml and sucrose at a concentration of 80mg per ml. The latex particle concentration is approximately 85mg per ml.

The reagent is deposited onto the mixing portion surface of a polypropylene-laminated card test stick in volumes of 1.25µl (4 spots providing a total of 5µl per test stick). This is done using a syringe pump system and an X-Y table to allow for precise positioning. The resulting stick is as seen in Figure 1. The sticks are provided in strip-arrays of 10, partially joined to allow easy separation, as seen in Figure 1.

The kit also includes positive and negative control sticks. Human serum, demonstrated to contain specific anti-*H*. *pylori* antibody (positive) or demonstrated not to contain specific anti-*H. pylori* antibody (negative), is diluted and sucrose added to provide a concentration of 80mg per ml.

The positive or negative control serum is deposited onto the mixing portions of respective positive or negative control sticks in volumes of 1.25µl (4 spots, a total of 5µl per stick). The sticks are clearly marked as positive or negative controls, but are otherwise substantially identical to the test sticks.

The control reagents can be reconstituted on a test surface using, for example, distilled water or aqueous buffers (such as phosphate-buffered saline), and then processed with a test stick in the usual way as if it were a normal sample liquid.

The spots of test and control reagents are dried on the card surface using a combination of warm air and infrared energy on a moving conveyor belt. The test stick arrays are packed in moisture impermeable, foil-laminate pouches.

The kit also comprises a test card, of known conventional type, upon which to perform the assay.

### Example 2

### Lancefield Streptococcal Grouping Kit

An embodiment of the present invention provides a Streptococcus grouping kit which can be used in conjunction with nitrous acid or enzyme extraction procedures for rapid identification of β-haemolytic streptococci of Lancefield types A, B, C, D, F and G. Differentiation between the various Lancefield groups may be desirable for clinical treatment and/or for epidemiological purposes.

The kit comprises six foil pouches, each containing 6 arrays of 10 test reagent sticks, substantially as shown in Figure 1. Each of the six pouches contains sticks having test reagents specific for one of the six Lancefield group antigens A, B, C, D, F or G. The test reagent comprises dried, pre-dosed blue monodisperse latex particles sensitised with rabbit antibody directed against the appropriate antigen. The kit thus comprises 60 test sticks for each Lancefield group.

The kit further comprises:- 3 strips of 10 sticks pre-dosed with positive polyvalent control reagent (dried antigen extract from group A, B, C, D, F and G Streptococci); white disposable test cards which provide a test surface for supporting the sample liquid; a plastics clip for re-sealing the foil pouches once opened; and instructions for use. As in Example 1, the test and control reagents are provided in an array of four dried drops, each originally of 1.25µl volume.

The kit may be produced as follows:
Specific rabbit hyperimmune anti-streptococcal antisera are produced by standard techniques. Lancefield group antisera A, B, C, D, F, and G are produced from separate groups of rabbits and minor cross reactions occurring with other bacteria are absorbed out. The antibody in the sera is partially purified by ammonium sulphate precipitation and dialysis against phosphate buffered saline pH 7.3.

Blue latex particles of diameter 300 - 400nm are suspended in phosphate buffered saline to make a 1% concentration. The temperature of the particle suspension is raised to 55°C in a water bath and the purified antibody is added. After a period of 30 minutes incubation the vessel is removed from the water bath and the latex suspension is centrifuged at 4000rpm to sediment the particles. The sensitised latex is then re-suspended at a concentration of 10% in phosphate buffered saline containing 1% BSA and 6% sucrose.

After filtration the suspension is spotted onto cards or sticks using an automated microdosing syringe system in volumes of 5µl per test (4 x 1.25µl spots).

The spots are dried at a temperature of 60°C and when cool the sticks are placed in a moisture impermeable foil pouch and a silica gel sachet added to remove any moisture which may enter at subsequent pouch openings.

### Test Procedure

1. Using known enzymatic or acid extraction methods (Maxted 1948 Lancet (ii) 255-256, Ederer *et al*, 1972 Appl. Microbiol. 23, 285-288; Lancefield 1938 Proc. Soc. Exp. Biol. 38, 473-478) an antigenic extract is prepared from a fresh culture of the organism (preferably grown on a blood agar plate overnight at 37°C). The reagents for performing the antigen extraction are included in the kit in some embodiments.
2. Using a pasteur pipette a 50 µl drop of sterile saline is added to a small circle at the base of an oval reaction area marked on the test card.
3. A positive control stick is removed from the pouch by tearing one off from the others on the strip, taking care to avoid touching the area where the dried reagents are located (if testing more than one reagent remove the appropriate number of sticks).
4. The stick is positioned so that the coloured spots of reagent are at the bottom and the stick placed on the test card with the spots touching the liquid. The stick is then pushed down so that it bends at the hinge. The stick is moved in a circular manner for 10 seconds to rehydrate the dried antigen extract.
5. The required number of test reagent sticks are removed from the pouch by tearing them away from the others on the strip, taking care to avoid touching the area where the spots of reagent are located. The stick is positioned so that the coloured spots are at the bottom, and then placed on the card with the spots touching the freshly prepared antigen suspension. The stick is then pushed down so that it bends at the hinge and the mixing portion used to mix the antigen suspension until the dried latex reagent is fully rehydrated and homogeneous.
6. The card is gently rocked and assessed for agglutination within the 1 minute test time.

The positive control must show agglutination with the dried test reagent within 1 minute. The test result is positive for the sample when agglutination occurs with one grouping reagent, or when one grouping reagent gives a substantially stronger reaction than the other five, within one minute. A negative result is obtained if no agglutination occurs and a smooth even blue suspension remains after 1 minute. Reactions occurring after 1 minute are to be ignored.

Those skilled in the art will appreciate that kits using the test sticks of the invention may be prepared in a manner which differs from that described above without departing from the invention. For example, the following parameters may be varied without substantially affecting the performance of the invention.

Latex particle sizes may vary from 100 to 900nm diameter, with alternative colours or even natural white colour.

Antibodies from other species such as goat, sheep, horse etc. or monoclonal antibody may be used. Antibody may be purified by a variety of techniques such as ion-exchange chromatography, protein A affinity chromatography etc.

The sensitisation buffer may be one of a number of suitable types including borate, glycine and Tris and the pH thereof may vary between pH 6 and 8.5, and the ionic strength may vary between 0.01 and 1 molar. The sensitisation temperature may vary between 4°C and 60°C. At high temperatures (eg. 60°C) the reaction may reach equilibrium within 10-15 minutes, whereas at low temperatures (eg. 4°C) it may be necessary to leave the reaction mixture for up to 18-24 hours. Further washes may be used to remove additional unbound antibody.

The BSA concentration in the resuspended particle mixture may be varied form 0.05 - 3% or more. The reagent concentration used for spotting may be varied between 1 % and 20% solids. Spotting volumes may be varied to suit concentration and reaction visibility requirements. Drying temperatures and times can be varied to suit air flow and evaporation rate.

## Claims

1. An assay device for testing for the presence of an analyte in a sample liquid supported on a test surface, the device comprising a test stick having a handle portion to be held by a person performing the test and a mixing portion to contact the sample liquid, the mixing portion bearing a dried, pre-dosed reagent for the test, the reagent being deposited on the mixing portion as a plurality of closely-spaced spots, such that contacting the mixing portion of the test stick with the sample liquid reconstitutes the test reagent, wherein the mixing portion is configured to provide a mixing surface to mix the reconstituted test reagent with the sample liquid on the test surface.

2. An assay device according to claim 1, wherein the dried reagent is particulate and the test result is revealed by the occurrence of, or inhibition of, agglutination of the particles following mixing with sample liquid.

3. An assay device according to claim 1 or 2 wherein the test stick comprises a strip of card, having a foldable tip.

4. An assay device according to any one of the preceding claims, wherein there is provided a line of weakness between the mixing and handle portions.

5. An assay device according to any one of the preceding claims, wherein the test reagent comprises an antibody specific for a Lancefield group-specific streptococcal antigen.

6. An assay kit comprising a plurality of test sticks in accordance with any one of the preceding claims.

7. An assay kit according to claim 6 comprising a plurality of disposable test sticks in the form of a "tear off" array of card strips.

8. An assay kit according to claim 6 or 7, further comprising one or more of the following: a test stick bearing a dried, pre-dosed positive or negative control reagent; reagents for preparing an extract of Lancefield group-specific antigen from Streptococci; and a test card providing a test surface for supporting the sample liquid during the assay.

9. A method of testing for the presence of an analyte in a sample liquid supported on a test surface, comprising using a test stick having a handle portion to be held by a person performing the test and a mixing portion bearing a dried, pre-dosed reagent for the test, the reagent being deposited on the mixing portion as a plurality of closely-spaced spots, such that the mixing portion of the test stick is contacted with the sample liquid so as to reconstitute the test reagent, and moving the mixing portion relative to the test surface so as to cause mixing of the reconstituted reagent with the sample liquid.

## Patentansprüche

1. Testvorrichtung zum Testen des Vorhandenseins eines zu bestimmenden Stoffs in einer Probenflüssigkeit, die von einer Testfläche getragen wird, wobei die Vorrichtung einen Teststab aufweist, der einen Griffabschnitt hat, der von einer den Test durchführenden Person zu halten ist, sowie einen Mischabschnitt, für einen Kontakt mit der Probenflüssigkeit, wobei der Mischabschnitt ein getrocknetes, vorher dosiertes Reagens für den Test trägt, wobei das Reagens auf dem Mischabschnitt als eine Vielzahl von Punkten in geringem Abstand zueinander so angeordnet ist, dass durch den Kontakt des Mischabschnitts des Teststabs mit der Probenflüssigkeit das Testreagens erstellt wird, wobei der Mischabschnitt so konstruiert ist, dass er mit einer Mischfläche versehen ist, um das wiederhergestellte Testreagens mit der Probenflüssigkeit auf der Testfläche zu mischen.

2. Testvorrichtung nach Anspruch 1, bei der das getrocknete Reagens partikelartig ist und das Testergebnis durch das Auftreten oder die Hemmung einer Agglutination der Partikel nach dem Mischen mit der Probenflüssigkeit offenbart wird.

3. Testvorrichtung nach Anspruch 1 oder 2, bei der der Teststab einen Kartenstreifen mit faltbarer Spitze aufweist.

4. Testvorrichtung nach einem der vorigen Ansprüche, bei der eine Sollbruchlinie zwischen dem Mischabschnitt und dem Griffabschnitt vorgesehen ist.

5. Testvorrichtung nach einem der vorigen Ansprüche, bei dem das Testreagens einen Antikörper enthält, der spezifisch für ein Streptokokken-Antigen ist, das spezifisch für die Lancefield-Gruppe ist.

6. Testsatz, der eine Vielzahl von Teststäben nach einem der vorigen Ansprüche enthält.

7. Testsatz nach Anspruch 6, der eine Vielzahl von Einweg-Teststäben in Form einer "Abreiß"-Anordung von Kartenstreifen aufweist.

8. Testsatz nach Anspruch 6 oder 7, der weiterhin eines oder mehrere der folgenden Elemente aufweist: Einen Teststab, der ein getrocknetes, vorher dosiertes positives oder negatives Kontrollreagens trägt, Reagenzien zum Herstellen eines Extrakts eines Streptokokken-Antigens, das spezifisch für die Lancefield-Gruppe ist, sowie eine Testkarte, die eine Testfläche zur Verfügung stellt, um die Probenflüssigkeit während des Tests zu tragen.

9. Verfahren zum Testen des Vorhandenseins eines zu bestimmenden Stoffs in einer Probenflüssigkeit, die von einer Testfläche getragen wird, das Folgendes umfasst: Die Verwendung eines Teststabs, der einen Griffabschnitt, der von einer den Test durchführenden Person zu halten ist, sowie einen Mischabschnitt, der ein getrocknetes, vorher dosiertes Reagens für den Test trägt, wobei das Reagens als eine Vielzahl von Punkten in geringem Abstand zueinander auf dem Mischabschnitt angeordnet wird, so dass der Mischabschnitt des Teststabs in Kontakt mit der Probenflüssigkeit tritt, um das Testreagens zu erstellen, sowie das Bewegen des Mischabschnitts bezüglich der Testfläche, um ein Mischen des wiederhergestellten Reagens mit der Probenflüssigkeit zu verursachen.

## Revendications

1. Dispositif de test pour déterminer la présence d'un analyte dans un échantillon liquide supporté sur une surface de test, le dispositif comprenant un bâton test ayant une portion manche destinée à la préhension par une personne réalisant le test et une portion de mélangeage pour mise en contact avec l'échantillon liquide, la portion de mélangeage étant munie d'un réactif de test séché, pré-dosé, le réactif étant déposé sur la portion de mélangeage sous forme d'une pluralité de points rapprochés, de sorte que la mise en contact de la portion de mélangeage du bâton test avec l'échantillon liquide reconstitue le réactif de test, dans lequel la portion de mélangeage est configurée pour fournir une surface de mélangeage pour mélanger le réactif de test reconstitué avec l'échantillon liquide sur la surface de test.

2. Dispositif de test selon la revendication 1, dans lequel le réactif séché est particulaire et le résultat du test est révélé par la survenance de, ou l'inhibition de, l'agglutination des particules consécutivement au mélangeage avec l'échantillon liquide.

3. Dispositif de test selon la revendication 1 ou 2, dans lequel le bâton test comprend une bande de carton ayant un bout pliable.

4. Dispositif de test selon l'une quelconque des revendications précédentes, dans lequel une ligne de faiblesse est présente entre les portions manche et de mélangeage.

5. Dispositif de test selon l'une quelconque des revendications précédentes, dans lequel le réactif de test comprend un anticorps spécifique d'un antigène spécifique d'un streptocoque du groupe Lancefield.

6. Kit de test comprenant une pluralité de bâtons tests selon l'une quelconque des revendications précédentes.

7. Kit de test selon la revendication 6, comprenant une pluralité de bâtons tests jetables sous forme d'un ensemble de bandes de carton détachables.

8. Kit de test selon la revendication 6 ou 7, comprenant en outre un ou plusieurs des éléments suivants : un bâton test portant un réactif contrôle positif ou négatif séché, pré-dosé ; des réaetifs pour préparer un extrait d'antigène spécifique de streptocoques du groupe Lancefield ; et un carton de test fournissant une surface de test pour supporter l'échantillon liquide pendant le test.

9. Procédé pour déterminer la présence d'un analyte dans un échantillon liquide supporté sur une surface de test, comprenant l'utilisation d'un bâton test ayant une portion manche destinée à la préhension par une personne réalisant le test et une portion de mélangeage munie d'un réactif de test séché, pré-dosé, le réactif étant déposé sur la portion de mélangeage sous forme d'une pluralité de points rapprochés, de sorte que la portion de mélangeage du bâton test cst mise en contact avec l'échantillon liquide de manière à reconstituer le réactif de test, et le déplacement de la portion de mélangeage par rapport à la surface de test pour permettre de mélanger le réactif test reconstitué avec l'échantillon liquide.
